(19) **Europäisches Patentamt**
**European Patent Office**
**Office européen des brevets**

(11) **EP 2 294 093 B1**

(12) **EUROPEAN PATENT SPECIFICATION**

(45) Date of publication and mention
of the grant of the patent:
**19.04.2017 Bulletin 2017/16**

(21) Application number: **09761503.3**

(22) Date of filing: **12.06.2009**

(51) Int Cl.:
*C08B 37/00* (2006.01)  *C12P 19/18* (2006.01)
*C07H 3/06* (2006.01)  *A23K 20/163* (2016.01)
*A23L 29/244* (2016.01)  *A23L 29/30* (2016.01)
*A23L 33/10* (2016.01)  *A23L 33/21* (2016.01)

(86) International application number:
**PCT/EP2009/004222**

(87) International publication number:
**WO 2009/149940 (17.12.2009 Gazette 2009/51)**

(54) **PROCESS FOR PRODUCING A FRUCTOOLIGOSACCHARIDE COMPOSITION**

HERSTELLUNGSVORGANG EINER FRUCTOOLIGOSACCHARID-ZUSAMMENSETZUNG

PROCÉDÉ DE PRODUCTION D'UNE COMPOSITION DE FRUCTO-OLIGOSACCHARIDES

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **12.06.2008 EP 08010679
13.06.2008 EP 08010793**

(43) Date of publication of application:
**16.03.2011 Bulletin 2011/11**

(73) Proprietor: **Tiense Suikerraffinaderij N.V.
1150 Brussel (BE)**

(72) Inventors:
• **DE LEENHEER, Lena
B-3080 Tervuren (BE)**
• **FROONINCKX, Kim, Martha, Jozefa
B-3000 Leuven (BE)**
• **HÉROUFOSSE, François, Alice, Alphonse
B-1457 Nil-Saint-Vincent (BE)**

(74) Representative: **Koster, Nico
Tiense Suikerraffinaderij N.V.
Patent Department
Aandorenstraat 1
3300 Tienen (BE)**

(56) References cited:
**EP-A- 0 301 628    WO-A-2007/061918
US-A- 4 734 402**

• HANG Y D ET AL: "Optimization of enzymatic
production of fructo-oligosaccharides from
sucrose" LEBENSMITTEL-WISSENSCHAFT AND
TECHNOLOGIE, vol. 29, no. 5-6, 1996, pages
578-580, XP002423975 ISSN: 0023-6438
• SANTOS ANDRELINA M P ET AL: "Synthesis of
fructooligosaccharides from sucrose using
inulinase from Kluyveromyces marxianus"
FOOD TECHNOLOGY AND BIOTECHNOLOGY,
vol. 45, no. 2, April 2007 (2007-04), pages 181-186,
XP009107555 ISSN: 1330-9862
• STOLL T ET AL: "Process for the recovery of a
carotene-rich functional food ingredient from
carrot pomace by enzymatic liquefaction."
INNOVATIVE FOOD SCIENCE AND EMERGING
TECHNOLOGIES, vol. 4, 2003, pages 415-423,
XP002539780 CORRESPONDENCE (REPRINT)
ADDRESS, A. SCHIEBER, INST. OF FOOD TECH.,
SECT. PLANT FOODSTUFF TECH., HOHENHEIM
UNIV., D-70599 STUTTGART, GERMANY. TEL.
+49-711-459-3125. FAX +49-711-45

EP 2 294 093 B1

**Description**

The invention relates to a process for the production of a fructooligosaccharide, to a fructooligosaccharide composition and its use.

Fructooligosaccharides as such are known, and find increasing use in a.o. foodstuffs, wherein they may be used in for example their quality of supplying non-sucrose sweetness, as fat replacer, as texturizer, or for their prebiotic properties.

A characteristic of fructooligosaccharides is that they fit in a profile of natural and healthy eating and living. It is however observed that the property profile of fructooligosaccharides can in this respect be improved even further, and this aim represents the objective of the present invention.

The said objective is achieved in the provision of a process for the production of a fructooligosaccharide (FOS) composition (FC) on industrial scale, comprising the steps of:

> a) providing a raw material containing sucrose;
> b) bringing the raw material together with an enzyme to form a mixture, whereby the enzyme has an endo-inulinase activity and is in free, non-immobilized form;
> c) exposing the mixture to conditions whereby FOS-forming can take place for an amount of time such that the FOS in the mixture constitutes at least 45 wt.% of the total amount of carbohydrates in the mixture, thereby forming the FOS composition; wherein the solids content of the mixture lies between 55°Bx and 80°Bx and the temperature lies between 55°C and 76°C;
> d) optionally deactivating the enzyme;
> e) recovering the FOS composition, wherein in step a) the amount of processed sucrose lies between 500 and 500,000 kg per 24 hours.

An advantage of the process according to the invention is that it may be implemented in a manner that satisfies all major standards referring to the production of Organic food ingredients. The FC thus produced can accordingly be marketed as Organic ingredient and so no longer represents a hindering factor in the preparation of all-Organic foodstuffs but may be readily incorporated therein and can confer sweetness, texture and prebiotic properties thereto.

In Japanese Patent application 62-295299 (publication number 1-137974), the enzymatic preparation of a FC from sucrose is disclosed. The enzyme used must be immobilised.

In "The Production of Fructooligosaccharides from Inulin or Sucrose Using Inulinase or Fructosyltransferase from Aspergillus ficuum", Barrie E. Norman & Birgitte Højer-Pedersen, Denpun Kagaku vol 36, No. 2, pp 103-111 (1989), the preparation on laboratory scale of a FC from longer-chain inulin or from sucrose is disclosed within the framework of basic research into enzymes and their workings.

In "Synthesis of Fructooligosaccharides from Sucrose Using Inulinase from Kluyveromyces marxianus", Andrelina M.P. Santos & Francisco Maugeri, Food Technol. Biotechnol. 45 (2) pp. 181-186 (2007), the preparation of a FC is disclosed. The preparation is enzymatic; the enzyme is immobilised in calcium alginate.

In EP-A-0 889 134 a process for producing a specific β-fructofuranisodase is disclosed, comprising the steps of cultivating a GMO (host cell) that was transformed via a recombinant plasmid.

Fructooligosaccharide (FOS) belongs to the compounds known as inulin. Inulin is a generic term that relates to a carbohydrate material consisting mainly of fructose moieties linked via β(2→1) type fructosylfructose links, with optionally a glucose starting moiety. Inulin is usually polydisperse, i.e. a mixture of compounds of various chain lengths whereby the degree of polymerisation (DP) of the individual compounds can range from 2 to 100 or higher. The term fructooligosaccharide - abbreviated as FOS - thus indicates a specific form of an inulin material, either monodisperse or polydisperse, whereby the DP of the individual compounds ranges from 2 to 10, in practice often from 2 to 9, or from 2 to 8 or from 2 to 7. Commercially available FOS is usually a polydisperse material having a number-averaged degree of polymerisation ($\overline{DP}$) of about 2 to 4.

In practice, FOS is also referred to as oligofructose. As used herein, the terms fructooligosaccharide and oligofructose are considered to be synonyms.

According to the invention, the FOS is part of a composition. As understood herein, a FOS composition means a composition that contains FOS - either monodisperse or polydisperse - and that furthermore may contain other compounds. Examples of such other compounds are: water, sucrose, fructose, glucose, inulin compounds other than FOS, maltose, organic salts, and inorganic salts. It is noted, however, that as used in the context of the present invention and when the FOS composition is intended for consumption by a human or an animal, the term FOS composition is to be regarded as an ingredient for a foodstuff rather than as a foodstuff itself. The creation of a foodstuff is thus a further step whereby the FOS composition serves as a food ingredient.

The process according to the invention is executed on industrial scale. It is well-known that it is no trivial matter to transfer laboratory findings into large-scale practice; in particular, it is known that in doing so many problems could arise and may need to be solved. Examples of such problems in the field of carbohydrate processes include scaling, turbidity, foaming, contamination with microbiological pathogens, and the need for purification steps. As meant herein, the term industrial scale means that the process is carried out in an installation that is able to process at least 500 kg of raw materials per 24 hours of operation; preferably this amount is between 1,000 and 1,000,000 kg per 24 hours. In a main embodiment, the process according to the invention relates solely to the preparation of a FOS composition: in this main embodiment the process is not combined with the preparation of another foodstuff; also preferably, the FOS composition is not prepared *in-situ* in an existing food product but rather is a food ingredient.

In step a) of the process according to the invention, a raw material is provided. This raw material should contain, or preferably consist essentially of, sucrose. In one embodiment the sucrose is Organic sucrose.

The terms 'consist(ing) essentially of or 'essentially only' and equivalents have, unless noted otherwise, in relation to a composition the usual meaning that in addition to compounds that are mandatory other compounds may also be present, provided that the essential characteristics of the composition are not materially affected by their presence.

Organic sucrose is as such known, and commercially available. One known source of Organic sucrose is Organic sucrose from sugar canes, supplied by Candico® (Belgium) and typically supplied in the form of solid crystals. Preferably, the raw material is certified according to at least one of the regulations (EU) or acts (US) relating to Organic practices currently in force. If the raw materials for the present Organic process contain other materials besides sucrose, then also these other raw materials should be allowable under Organic process practices.

In step b) of the process according to the invention, the raw material is brought together with an enzyme, thereby forming a mixture.

The purpose of the enzyme is to catalyse the formation of FOS from sucrose; this may be achieved by selecting an enzyme having fructosyltransferase activity. Such enzymes are as such known, for instance as categorised under enzyme category number EC 2.4.1.99 or EC 2.4.1.9. An early disclosure of such an enzyme is in "The Production of Fructooligosaccharides from Inulin or Sucrose Using Inulinase or Fructosyltransferase from Aspergillus ficuum", Barrie E. Norman & Birgitte Højer-Pedersen, Denpun Kagaku vol 36, No. 2, pp 103-111 (1989). Furthermore, it is known that some $\beta$-fructofuranosidases, i.e. enzymes categorised under EC 3.2.1.26, can also have fructosyltransferase activity .

It was found, surprisingly, that also enzymes having an endo-inulinase activity - such as enzymes classified under EC 3.2.1.7 - may in the presence of sucrose give rise to the formation of FOS, in particular if they act in a mixture having a high solids content of 60 °Bx or h igher. The invention thus relates to a method for the enzymatic preparation of a fructooligosaccharide composition from sucrose wherein the enzyme contains or even consists essentially of an endo-inulinase.

One example of a preferred enzyme for use in step b) of the invention is the endo-inulinase Novozyme 960 (supplier: Novozymes). Another example of a preferred enzyme for use in step b) of the invention is Pectinex Ultra SP-L (supplier: Novozymes).

In an embodiment of the invention, the enzyme used in step b) is an enzyme that also occurs as such in nature. In this embodiment, no genetic engineering activity has been undertaken to change the amino acid sequence of the enzyme, or of the underlying DNA that leads to the formation of the enzyme. It is furthermore preferred that no genetically modified organism (GMO) was used in order to prepare the enzyme on commercial scale, so that the enzyme is not made by a GMO. Similarly, it is preferred that no microorganism obtained through mutation, for example via purposeful exposure to chemicals or radiation, was used in the preparation of the enzyme used in the process according to the invention.

The enzyme may partly be used in immobilized form, but should also partly or preferably even wholly in non-immobilized form - whereby it is known that non-immobilized is also referred to as 'free'. If the enzyme is partly used in immobilised form, it is preferred that the immobilizing was done without the use of cross-linkers or other substances that are not allowed under Organic production practices. In a main embodiment of the invention, however, the enzyme is not immobilized at all.

The amount of enzyme needed in the process according to the invention depends on various as such known factors such as process temperature, amount of raw materials, pH, allowable process duration, and desired conversion rates. These and other relevant factors may be determined by the person skilled in the art following the generally accepted procedures in this technical field.

When forming the mixture, it is preferred - as is usual in this type of process - that water is present or added so that water becomes the continuous phase in the mixture. If so, then this may however also be done as part of subsequent step c).

In step c) of the invention, the mixture as formed in step b) is exposed to conditions whereby FOS-forming can take place for an amount of time such that a desired amount of FOS has been formed, thereby forming the FOS composition. When not already done in step b), it is preferred in step c) that water is added so that it becomes the continuous phase in the mixture. It may be helpful or even necessary to submit the mixture to a treatment such as an ultra-high temperature treatment (UHT); this may a.o. help to ensure that essentially no pathogens are contained or remain in the mixture. In a main embodiment of the invention the mixture is exposed to conditions whereby FOS-forming can take place for an amount of time such that the FOS in the thus-formed FOS composition constitutes at least 45 wt.% of the total amount of carbohydrates in the mixture. Preferably, the said amount of FOS is at least 50, 53, 55, 56, or even at least 57 wt.% of the total amount of carbohydrates in the mixture. Due to the circumstance that a certain amount of free glucose will typically be formed in the process, a limit may exist on the maximum amount of FOS obtainable in the mixture. Thus, the amount of FOS in the mixture at this stage of the process is preferably at most 65, 64 or 63 wt.% of the total amount of carbohydrates.

As is known, the unit '°Bx' - short for 'degrees Brix' - is a widely used term in the sugar industry to indicate solids content of a sugar solution, as derived from its refraction index. As used herein, the same method is used on samples of (Organic) sucrose or on FOS compositions. As is known, the numerical °Bx values are very close to, or even essentially identical to, weight percentages; thus in an alternative expression of the present invention all °Bx values as mentioned in the present description and claims are read as being weight percentages of dry matter.

The temperature in step c) is at least 55°C. Surprisingly, it was found that process step c) according to the invention can show unexpected exceptional results when the temperature in step c) lies between 60°C and 75°C and the solids content lies between 61 and 75°Bx. Contrary to the expectation that at such conditions of elevated temperature the FOS formation would deteriorate as compared to temperatures below 60°C, it was found that good or even improved FOS formation can take place.

Moreover, it was found that when the solids content of the mixture is above 55, 58, 60, 61, 62, 63 or 64°Bx, that then in the process according to the invention the conditions for growth of microorganisms are increasingly unfavourable. The said conditions can even be unfavourable to such an extent that there is no need for the use of processing aids aimed at suppressing the growth of microorganisms like bactericides. This characteristic of the process according to the invention is moreover instrumental in case of a highly efficient Organic implementation of it.

The enzyme or combination of enzymes as used in the process of the invention may be used partly in immobilized form. However, in the main embodiment of the process according to the invention it was found that the process can be operated in a particularly efficient manner if in step c) the enzyme is not immobilized at all. According to the invention, in step c) the solids content of the mixture lies between 55, 58, 60, 61, 62, 63 or 64 and 80, 78, 76, 75, 74, 73 or 72°Bx. The temperature then lies between 55, 56, 57, 58, 59, 60, 61, 62, 63, 64, or 65°C and 76, 75, 74, 73, 72 or 71°C.

When the mixture is in step c) exposed to conditions of temperature and concentration as indicated above, the enzymatic action will lead to the formation of Organic FOS; free glucose may be - and usually is - formed at the same time. It may be useful or even necessary to control the pH during the execution of step c). The precise range of pH is, as the skilled person knows, dependent on several factors such as in particular the choice and characteristics of the enzyme as used. The controlling of the pH may be executed by means that are as such known, whereby preferably care should be taken that any compounds used to this end are acceptable within the framework of Organic production. Examples of acidifying compounds that are acceptable in at least one Organic regulation/law/standard are citric acid and sulphuric acid. The duration of execution of step c) according to the invention is mainly chosen in function of the amount of FOS that is desired. As the skilled person knows, this duration is often in the range between 1 and 72 hours, preferably between 5 and 50 hours, more preferably between 12 and 36 hours, during which the FOS composition (FC) is formed.

Upon completion of step c) of the invention, it may be desirable to ensure that the enzyme is deactivated. If this is the case, then an enzyme deactivating step d) may be implemented. The implementation of step d) is also preferred due to the circumstance that the enzyme used is at least partly not immobilized. The deactivation of the enzyme may be achieved by methods that are as such known and may differ for each specific type of enzyme. An example of such a method of deactivation is an increase in temperature - to a level of for example about 80, 85 or 90°C - followed by a residence time of between 5 and 30 minutes at such an increased temperature. A further benefit of exposure at such a temperature is that the amounts of any bacteria that may be present are reduced dramatically.

Subsequent to step c) - or possibly step d) - the FC is recovered in a recovery step e). The recovery step e) of the invention may optionally include the addition of compounds to the FC. It was found that when the FC is recovered as such - i.e. typically as aqueous solution - it is already suitable for subsequent use as ingredient in foodstuffs or in animal feed. It is also possible to utilize a subsequent processing step, whereby any such subsequent step is preferably acceptable within the framework of Organic (ingredient) production. Examples of such subsequent steps are:

1. A sterilising step, for example by means of UHT;
2. A filtering step wherein the FC is passed through filters; said filters may optionally have as purpose to help ensuring that the FC becomes or remains sterile;
3. A heating step, for example to a temperature lying between 85°C and the boiling point of the FC or higher, e.g. with the aim of evaporating a portion of the water present in the FC.

Preferably and in view of its preferred Organic character, in none of the steps of the process according to the invention use is made of one or more of the following substances: anti-scaling agent, anti-foaming agent, bactericide, biocide, and flocculant. Moreover, it is preferred that chromatographic separation techniques are not used in any step of the process according to the invention. Similarly, it is preferred that ion-exchange resins are not used in any step of the process according to the invention. In general, refining steps are preferably not used. It was found in particular that the operation of the process at high temperatures and at high solids content is instrumental in enabling to forego on the abovementioned substances and techniques when implementing the process of the invention on industrial scale.

It is noted that the non-use of the substances and techniques as mentioned above is no trivial matter, as these are substances and techniques that are quite common in the industry of FOS preparation. The non-triviality thus holds in particular for the implementation on industrial scale of the process of the invention. In this context the person skilled in the art may, via routine experimentation, vary process conditions such as in particular solids content and temperature within the ranges and preferred ranges as given above so as to arrive at conditions whereby indeed the abovementioned absence of certain substances and techniques is possible on industrial scale.

According to the invention therefore, the amount of processed sucrose in step a) lies between 500 and 500,000 kg per 24 hours. In this way the process according to the invention can convert between 500 kg and 500,000 kg of sucrose per 24 hours into a FOS composition.

When executing a process for the preparation of a FOS composition, it will be appreciated that the person skilled in the art has at his disposal a number of parameters that may be varied in order to optimise the process and the resulting FOS composition. Among the most important of these parameters rank, as the skilled person knows: the temperature at which the synthesis is carried out, the solids content of the mixture in steps b) and c), the duration of the enzymatic reaction, and the amount of enzyme that was added.

It was found, surprisingly, that certain selections of these parameters can lead to specific FOS compositions having desirable characteristics. In particular, the combination of:

- relatively high values of solids content and temperature along the guidelines as given above;
- the use of a free, non-immobilized enzyme, whereby preferably the said enzyme was not prepared using GMO's; and
- performing routine experiments to reduce the amount of enzyme as much as possible while still obtaining at least acceptable conversion rates,

can lead to FOS composition having one or more of the following characteristics:

- a high amount of FOS having a degree of polymerisation (DP) of 3, relative to the total amount of fructooligosaccharides; and/or
- a low amount of FOS having a DP of 7 or higher, relative to the total amount of fructooligosaccharides; and/or
- a high amount of FOS in the composition as a whole, while still retaining a relatively high amount of sucrose.

It is preferred that a portion of at most 3, more preferably at most 2, 1, or even 0.50, 0.40, 0.30 or 0.20 wt.% of the carbohydrates in the FC consists of oligosaccharides that have a degree of polymerisation (DP) of 7 or more. In this embodiment, an amount at or above 0.01, 0.05 or 0.10 wt% of oligosaccharides that have a degree of polymerisation (DP) of 7 or more may be present, while respecting the upper limits as given before.

It was found that the process according to the invention can lead to a FC wherein a high percentage of the fructooligosaccharides in the FC have a degree of polymerisation (DP) of 3 as measured in HPLC. The dominant compound having a DP of 3 in the FC prepared according to the invention is 1-kestose. Surprisingly, it was found that such a FC having a high amount of FOS with DP3 may be achieved through the use of enzymes that are known to occur as such in nature.

The process of the invention therefore produces a FC wherein:

• at least 56 wt.% of the fructooligosaccharides in the FC have a DP of 3 as measured in HPLC; and
• the FC is obtained from sucrose in an enzymatic process, whereby no enzyme is used that is obtained from a genetically modified organism.

Preferably, a portion of at most 2 wt.% of the carbohydrates in the FC consists of oligosaccharides that have a degree of polymerisation (DP) of 7 or more. In further preferred embodiments, the amount of FOS having DP 3, calculated on the total amount of FOS in the FC and as measured in HPLC, is at least 57, 58, 59, 60, 61, 62, 63, 64, 65, 66, 67, 68, 69, or even at least 70 wt.%. It was found that the circumstance that at least 56 wt.% of the fructooligosaccharides has a DP of 3 has the advantage that the solubility of the FC in water increases and/or that the tendency of the FC to show turbidity is decreased.

Preferably, the carbohydrates in the FC contain at least 11 wt.%, preferably at least 11.5, 12.0, 12.5, 13.0, 13.0 or even at least 14 wt.% of sucrose - this although the FC should be obtained from sucrose in an enzymatic process, whereby no GMO and preferably no mutated microorganism was used to prepare the enzyme; the enzyme was preferably in free, non-immobilized form. This has the advantage that the amount of carbohydrates that are not FOS, and which are generally less desirable, is reduced. In this embodiment, it is preferred that the FC has not been subjected to separation techniques such as chromatographic separation to remove carbohydrates from the FC. The process of the invention also produces a FC wherein:

- at least 55 wt.% of the carbohydrates in the FC are fructooligosaccharides; and
- at least 11 wt.% of the carbohydrates in the FC consists of sucrose; and
- the FC is obtained from sucrose in an enzymatic process, whereby no enzyme is used that is obtained from a genetically modified organism.

In this main embodiment, the carbohydrates in the FC contain a high amount of at least 55 wt.%, preferably at least 56, 57, 58, 59 or even at least 60 wt.% of fructooligosaccharides; at the same time, the carbohydrates in the FC contain at least 11 wt.%, preferably at least 11.5, 12.0, 12.5, 13.0, 13.0 or even at least 14 wt.% of sucrose - this although the FC should be obtained from sucrose in an enzymatic process, whereby no GMO and preferably no mutated microorganism was used to prepare the enzyme; the enzyme was preferably in free, non-immobilized form. This has the advantage that the amount of carbohydrates that are not FOS, and which are generally less desirable, is reduced. In this embodiment, it is preferred that the FC has not been subjected to separation techniques such as chromatographic separation to remove carbohydrates from the FC.

The process according to the invention can yield an Organic product. This implies that the choice and execution of the process steps according to the invention are then such that it complies with the relevant regulations.

As used herein, the term 'organic' means relating to organic chemistry; the term 'Organic' - i.e. with capital 'O' - means relating to agriculture, processes and products fulfilling certain criteria and typically aimed at integrating cultural, biological, and mechanical practices that foster cycling of resources, promote ecological balance, and conserve biodiversity.

The FOS produced according to the invention can be Organic. As is known, the characterisation "Organic" of a process or a product is in many countries a legally protected term and carries with it a series of demands placed on the process or product. Examples of such demands in agriculture are:

- The avoidance / absence of certain or all herbicides and pesticides. Typically, synthetically prepared herbicides and pesticides are prohibited except when placed on a positive list.
- The avoidance / absence of certain or all fertilizers, combined with active soil fertility management. Typically, synthetically prepared fertilizers are prohibited except when placed on a positive list.
- Maximizing the use of renewable resources;
- Multiannual crop rotation;
- The absence/non-use of genetically modified organisms;
- The avoidance or non-use of certain processing aids or processing steps, e.g. in the obtaining of an ingredient from a raw material. For example, the obtaining of Organic sucrose from Organic sugar cane or Organic sugar beets is done a.o. without the use of certain refining steps such as chromatographic separation and preferably also the use of ion exchange resins.

Furthermore, Organic processed products should preferably be produced by the use of processing methods which guarantee that the Organic integrity and vital qualities of the product are maintained through all stages of the production chain. One example of a measure taken in this context is the avoidance of using enzymes that are made by or using GMO's.

As is clear from the above, the term Organic can be applicable to products and processes in a variety of stages and types, such as for example: a farming method, an agricultural crop, a process for obtaining an ingredient from an agricultural crop, the ingredient itself, a product composed of various ingredients, etc..

The term Organic thus has certain core elements that are universal, although depending on the type of process or product it relates to. Relating to processes for obtaining an ingredient from an agricultural crop, the meaning of Organic includes the use of Organic raw materials and the respecting of Organic manufacturing practices. Relating to ingredients obtained from an agricultural crop the meaning of Organic includes a certainty of the Organic nature of the raw materials used, the respecting of Organic manufacturing practices, and the absence of certain compounds such as herbicides and pesticides and preferably also fertilizers in the ingredient as supplied.

The meaning of the term Organic may have in confirmation of or addition to this certain country-specific elements, which are then explicitly incorporated into standards, laws, directives, regulations and the like. Two widely known implementations of the demands coming with the term Organic are: in Europe the European Regulation EC 834/2007 of 28 June 2007

(published on 20.07.2007 in the Official Journal of the European Union, OJ L189, as successor of Regulation 2092/91) and in the USA the US Organic Foods Production Act (OFPA). Preferably, the Organic FOS composition according to the invention complies with all applicable sections of the said European Regulations and/or the US OFPA. The Organic FOS composition according to the invention will thus also comply with the same, similar, or equivalent sections of future versions of the said regulations/Act.

In a related embodiment, the Organic FOS composition according to the invention is obtained by a process complying with all applicable sections of the said European Regulations and/or the US OFPA.

An Organic FOS composition (OFC) produced according to the invention will preferably, and in line with its Organic character, contain essentially no herbicides, pesticides, or fertilizers, except those that are allowed for use under Organic regulations or acts. More preferably an OFC produced according to the invention contains essentially no detectable amounts of herbicides, pesticides, or fertilizers at all.

In an embodiment of the invention, the process for the preparation of an OFC and also the OFC produced according to the invention are essentially free of prohibited substances appearing on the USA National List of Allowed and Prohibited Substances as published within the framework of the US National Organic Program (NOP), hosted by the Agricultural Marketing Service of the United States Department of Agriculture. In particular this relates to Part 205 of Title 7 (Agriculture) of the Code of Federal Regulations, specifically to §205.602, "Nonsynthetic substances prohibited for use in organic crop production." Moreover, it is preferred that any substances that carry over from the Organic sucrose crop into the process of the invention and possibly also into the OFC according to the invention appear on the applicable positive lists of the said National List of Allowed and Prohibited Substance, in particular §205.601" Synthetic substances allowed for use in organic crop production." §205.605 "Nonagricultural (nonorganic) substances allowed as ingredients in or on processed products labelled as "organic" or "made with organic (specified ingredients or food group(s))." ", and §205.606 "Nonorganically produced agricultural products allowed as ingredients in or on processed products labelled as "organic."

In a further embodiment of the invention, the process for the preparation of an OFC and also the OFC produced according to the invention comply with European Commission Regulation 207/93 of 29.01.1993 and amended on 26.02.1997 (with Regulation 345/97) and on 26.09.2000 (with Regulation 2020/2000). This regulation defines the content of Annex VI to the abovementioned Regulation 2092/91 and covers the ingredients and processing aids which may be used in the preparation of foodstuffs composed essentially of one or more ingredients of plant origin, referred to in Article 1 (1) (b) of this Regulation, with the exception of wines.

A FOS composition produced according to the invention may contain, as is typically the case for compositions recovered in an industrial processes, a small amount of impurities. As meant herein, the term impurity refers to compounds other than carbohydrates or water. In the case of an FC, the most important impurities are inorganic salts, organic acids and proteins. The total amount of impurities in the FC produced according to the invention can be in the range of 0.1 - 3 wt.% of the FC as a whole. If the FC according to the invention is an OFC, then it may be, and in particular if the process according to the invention uses Organic sucrose from sugar cane, that the amount of impurities lies between 0.5 and 5 wt.%; whereas in the case of refined sucrose as raw material the amount of impurities typically lies in the range between 0.1 or 0.2 and 0.5 wt.%.

If the FC produced according to the invention is an OFC and the raw material used to prepare the OFC contained or even consisted of Organic sucrose from sugar cane, then it was found that a mixture of various non-carbohydrate compounds that was contained in the Organic sucrose, and regarded therein as valuable since it elevates the Organic sucrose from being a mere supplier of 'dead calories', may also be contained at least partly in the OFC. The present invention therefore also relates to an FC that contains in total between 0.1 and 5 wt.% of one or more compounds selected from the group consisting of minerals, vitamins, amino acids and proteins. Preferably, the total amount of the said compounds is at least 0.2, 0.3, 0.5, 0.6, 0.7, 0.8, 0.9, or even at least 1 wt.%; this has the advantage that additional beneficial effects in taste and/or health may be present in or associated with the FC. Preferably, the total amount of the said compounds is at most 4.5, 4.0, 3.5, 3.0, 2.9, 2.8, 2.7, 2.6, 2.5, 2.4, 2.3, 2.2, 2.1, or even at most 2.0 wt.%.

It is preferred, in particular in the case when the FC is an OFC, that any minerals, vitamins, amino acids or proteins present were not added to the FC but rather were either already integrally contained in the raw materials used to prepare the (O)FC, in particular in the raw material Organic sucrose, or result from processing aids being used in the preparation of the (O)FC.

The amount of FOS in the FOS composition produced according to the invention preferably lies between 35 wt.% and 99 wt.%, more preferably between 40 wt.% and 98 wt.%, most preferably between 45 wt.% and 97 wt.%. If the FC should contain more FOS than was achieved in steps a) through d) according to the invention, then a subsequent step is necessary in order to increase the amount of FOS in the FC; and example of such a subsequent step is the - as such known - step of chromatographic separation. The FC contains essentially no pathogenic microorganisms and also essentially no Enterobacteriaceae. As meant herein, the qualification 'essentially no pathogenic microorganisms' means that in one (1) gram of the FC zero (0) pathogenic microorganisms (as expressed in CFU, colony-forming units) were detected. The absence of pathogenic microorganisms in one gram of sample is in particular true for *Staphilococcus aureus*, *Escherichia coli, Clostridium perfringens*, *Clostridium botulinum, Salmonella* and *Listeria.* As meant herein, the qualification 'essentially no Enterobacteriaceae' means that in one (1) gram of the FC zero (0) Enterobacteriaceae (as expressed in CFU, colony-forming units) were detected. For the Enterobacteriaceae this is preferably established by using the well-known evaluation on colour using VRBGA (Violet Red Bile Glucose Agar) and after 24 hours of incubation at 37°C.

More preferably, the FC produced according to the invention contains no Enterobacteriaceae after enrichment. As meant herein, enrichment means that a sample of material is first incubated under conditions that are chosen so that they are optimal for any Enterobacteriaceae present to grow and multiply. This is done by first bringing 25 grams of a sample of material together with 225 ml of Peptone water and letting this mixture incubate at a temperature lying between 35 - 37°C for 18 - 24 hou rs. Then, 1 ml of this mixture is added to 9 ml of an Enterobacteriaceae Enrichment (EE) broth, followed by incubation at a temperature lying between 35 - 37°C for 18 - 24 hours. This completes the enrichment. Subsequently, the EE broth sample is tested on the presence of Enterobacteriaceae using VRBGA. In this preferred embodiment of the invention, even after enrichment no Enterobacteriaceae are detected.

One favourable method of obtaining an Organic FOS composition (OFC) according to the invention starts from the raw material Organic sucrose, preferably prepared from Organic sugar cane or Organic sugar beet. Due to the characteristics of production of Organic sucrose, in particular the absence of certain or even all refining steps, this raw material can contain as already indicated above compounds other than sucrose that cause a characteristic colour and flavour; this holds in particular for Organic sucrose from sugar cane. As the same restrictions on refining that apply to an Organic process for the preparation of Organic sucrose also apply to an Organic process for the preparation of a FOS composition, it can be that the abovementioned compounds causing colour or flavour carry over into the OFC. Furthermore, a process for preparation of an OFC itself may introduce compounds other than FOS into the OFC. As a result of these factors, it was found that in an embodiment of the OFC according to the invention, the OFC has a colour lying between 30 and 20,000 Icumsa units, preferably between 75 and 15,000, more preferably between 100 and 10,000, in particular between 125 and 9,000 or between 150 and 8,000, and most preferably between 175 and 7,000. In this embodiment, but also according to the invention in a separate embodiment of the OFC produced according to the invention, the OFC has a conductivity lying between 30 and 5,000 $\mu$S/cm, preferably between 40 or 50 or 60 and 4,000, more preferably between 70 or 80 or 100 and 3,000 and most preferably between 125 or 150 or 175 and 2,000 $\mu$S/cm.

Icumsa units as indication for colour are widely known and used in the sucrose-producing industry. An authoritative test method for determining the Icumsa colour is method GS2/3-9 (2005), which is in essence a measurement of the absorption of an aqueous solution of sucrose at a wavelength on 420 nm; the absorption is then re-calculated into the Icumsa value. Low Icumsa values indicate a colourless/white colour; higher values are evidence of a product having a colour, which is often yellow to brownish. The method GS2/3-9 (2005) is also used herein for the determination of the colour of an OFC, with the following comments/modifications:

- The pH is regulated to 6 (not to 7 as in the standard);
- The formula for determining the Icumsa colour of a sample is:

$$Icumsa\,Colour \equiv \frac{100000 \times A}{Bx \times \rho \times b}$$

wherein $A$ is the absorption at 420 nm, $Bx$ is the solids content in degrees Brix, $\rho$ is the density of the sample, and $b$ is the length of the absorption path.

The conductivity of an OFC is determined on an aqueous OFC having a solids content of 28 °Bx, and expressed in microS iemens per centimetre ($\mu$S/cm).

The invention will be illustrated by means of the following examples, without being limited thereto.

Examples 1 - 3

In a laboratory experiment, Organic sucrose (supplier: Candico) from sugar cane origin was combined with water and an enzyme to form a mixture. The solids content was 72 °Bx. The enzyme was the endo-inulinase Novozyme 960, not immobilized. The activity of the enzyme was 470 U per gram. One unit (herein referred to as U, also known as EU) of an endoinulinase enzyme is the amount of enzyme that is capable of liberating 1 $\mu$mol reducing sugar per minute from an inulin sample (see also page 108 left column of "The Production of Fructooligosaccharides from Inulin or Sucrose Using Inulinase or Fructosyltransferase from Aspergillus ficuum", Barrie E. Norman & Birgitte Højer-Pedersen, Denpun Kagaku vol 36, No. 2, pp 103-111 (1989)). The amount of enzyme used in the mixture was 0.4 U per gram dry matter of raw material.

The mixture was brought to a pH of 6.25 by using $H_2SO_4$ and/or NaOH. The mixture was heated to a temperature as indicated in the table below, and left to react for 20 hours. During this time, the pH decreased very slightly to a level of about 6.12. An Organic FC (OFC) was formed. After the said 20 hours the reaction was stopped; this was done by briefly cooking the OFC in a microwave oven. No further refining steps or purification steps were performed on the OFC.

The OFC was analysed by means of HPLC. The results are summarized in Table 1 below. The OFC was also subjected to a bacteria count; no Enterobacteriaceae were found.

*Table 1*

| Example number | 1 | 2 | 3 |
|---|---|---|---|
| Temperature (°C) | 66 | 70 | 72 |
| Non-Fructooligosaccharides | | | |
| - Fructose | 3.1 | 4.7 | 4.7 |
| - Glucose | 24.1 | 22.2 | 20.4 |
| - Sucrose | 14.6 | 14.8 | 19.6 |
| Fructooligosaccharides | | | |
| - DP2 (other than Sucrose) | 0.7 | 0.7 | 0.5 |
| - DP3 | 37.5 | 37.5 | 39.2 |
| - DP4 | 18.0 | 18.2 | 14.2 |
| - DP5 and higher | 1.9 | 1.9 | 1.2 |
| → Total FOS | 58.2 | 58.3 | 55.3 |
| → % of FOS having DP3 | 64 | 64 | 71 |

*Legend to Table 1*

- The amounts of the various compound are given in weight percentage of total dry carbohydrate matter;
- The terms DP2, DP3, DP4 and DP5 indicate compounds having a degree of polymerisation of 2, 3, 4 and 5, respectively.

The Examples 1 through 3 clearly show that an OFC can be obtained, by means of a method respecting principles of Organic production and by using a non-immobilized endo-inulinase.

Example 4

In a pilot plant test, an Organic FC (OFC)was produced from a starting amount of 1000 kg of Organic sucrose from sugar cane (supplier: Candico). A mixture was created by combining the Organic sucrose with water and an enzyme. The amount of water was such that the solids content of the mixture was 60 °Bx. The enzyme (Novozyme 960) was used in a n amount of 0.280 U per gram of Organic sucrose. The enzyme was not immobilized. The pH was regulated to 6.2 by using $H_2SO_4$ and/or NaOH and the temperature was brought to 56°C. Under these conditions, the mixture was left to react for 20 hours, thereby forming an OFC. Subsequent to this, the reaction was slowed down to essentially standstill by bringing the pH of the OFC to 8. The OFC was then pushed through an $18\mu$ filter and the OFC concentrated through heating so as to bring the solids content to 75 °Bx and deactivate the enzyme. The OFC as then recovered had a colour of 350 lcumsa units and a conductivity of $400\mu S/cm$. No use was made of an anti-scaling agent, an anti-foaming agent, a bactericide, a biocide, or a flocculant. The composition of the OFC as analysed by means of HPLC is given in Table 2. The terms in Table 2 have the same meaning as in Table 1.

*Table 2*

| Example number | 4 |
|---|---|
| Temperature (°C) | 56 |
| Non-Fructooligosaccharides | |

(continued)

| Example number | 4 |
|---|---|
| - Fructose | 1.3 |
| - Glucose | 25.8 |
| - Sucrose | 13.2 |
| Fructooligosaccharides | |
| - DP2 (other than Sucrose) | 0.4 |
| - DP3 | 37.7 |
| - DP4 | 19.7 |
| - DP5 and higher | 1.9 |
| → Total FOS | 59.6 |
| → % of FOS having DP3 | 63 |

Example 4 clearly shows that also at conditions differing significantly from those in Examples 1 through 3, and in a process incorporating various subsequent treatments after initial FOS forming - although still according to the invention - an OFC may be prepared. Furthermore, this Example shows that the process according to the invention may indeed be successfully implemented on industrial scale.

Example 5: Comparative Experiment A

In two laboratory tests, a comparison was made between the use of an immobilized enzyme (Comparative Experiment A) and a free (i.e. non-immobilized) enzyme (Example 5). In both cases, the enzyme was Novozyme 960. The carrier for the immobilized enzyme was calcium alginate. In both cases, the raw material was Organic sucrose from sugar cane (supplier: Candico), and the amount of water was such that the solids content of the mixture was 60 °Bx. The free enzyme was used in an amount of 0.3 U per gram (dry matter) of Organic sucrose; the immobilized enzyme was used in an amount of 2.43 U per gram (dry matter) of Organic sucrose. The other conditions were as in Example 4. The composition of the resulting Organic FC was determined by means of HPLC and is summarized in Table 3, wherein the terms have the same meaning as in Table 1.

*Table 3*

| Comparative Experiment / Example number | A | 5 |
|---|---|---|
| Temperature (°C) | 56 | 56 |
| Non-Fructooligosaccharides | | |
| - Fructose | 1.8 | 1.1 |
| - Glucose | 30.1 | 27.0 |
| - Sucrose | 12.4 | 12.3 |
| Fructooligosaccharides | | |
| - DP2 (other than Sucrose) | 2.5 | 0.5 |
| - DP3 | 28.4 | 36.5 |
| - DP4 | 18.3 | 20.8 |
| - DP5 and higher | 6.5 | 1.9 |
| → Total FOS | 55.7 | 59.6 |
| → % of FOS having DP3 | 46 | 61 |

Example 5 according to the invention show that superior results may be obtained by a free enzyme, as the use of a free enzyme in Example 5 led to:

- a higher overall yield of FOS compounds;
- a higher amount above 56% of oligosaccharides having DP3, and
- a lower amount of FOS compounds having DP5 or higher,

when compared to the use of an immobilized enzyme in Comparative Experiment A.

Example 6

(not according to the invention)

In a laboratory experiment, Organic sucrose (supplier: Candico) from sugar cane origin was combined with water and an enzyme to form a mixture. The solids content was 59.7 °Bx. The enzym e was Pectinex Ultra SP-L (supplier: Novozymes). Although this enzyme has as its main application area pectin hydrolysis, it is known that it also has fructosyltransferase activity - see for example "Immobilization of pectinex ultra SP-L pectinase and its application to production of fructooligosaccharides" by Csanadi, Zs. and Sisak, Cs. in Acta Alimentaria Vol. 35 (2) pp 205-212 (2006). The amount of enzyme used in the mixture was 162 U per gram dry matter of raw material. The enzyme was non-immobilized (i.e. free).

The mixture was brought to a pH of 6.10 by using $H_2SO_4$ and/or NaOH. The mixture was heated to 56°C, and left to react for 20 hours. An Organic FC (OFC) was formed. After the said 20 hours the reaction was stopped; this was done by briefly cooking the OFC in a microwave oven. No further refining steps or purification steps were performed on the OFC.

The OFC was analysed by means of HPLC. The results are summarized in Table 4 below. The OFC was also subjected to a bacteria count; no Enterobacteriaceae were found.

Table 4

| Example number | 6 |
|---|---|
| Non-Fructooligosaccharides | |
| - Fructose | 2.1 |
| - Glucose | 26.1 |
| - Sucrose | 11.5 |
| Fructooligosaccharides | |
| - DP2 (other than Sucrose) | 0.7 |
| - DP3 | 37.1 |
| - DP4 | 20.8 |
| - DP5 and higher | 1.8 |
| → Total FOS | 60.3 |
| → % of FOS having DP3 | 61.5 |

Example 7

In a laboratory experiment, Organic sucrose from Organic sugar beet origin (supplier: Südzucker AG) was combined with water and an enzyme to form a mixture. The solids content was 60.0 °Bx. Th e enzyme was Novozyme 960. The amount of enzyme used in the mixture was 0.64 U per gram dry matter of raw material. The enzyme was non-immobilized (i.e. free).

The mixture was brought to a pH of 6.2 by using $H_2SO_4$ and/or NaOH. The mixture was heated to 56°C, and left to react for 15 hours. An Organic FC (OFC) was formed. After the said 15 hours the reaction was stopped; this was done by briefly cooking the OFC in a microwave oven. No further refining steps or purification steps were performed on the OFC.

The OFC was analysed by means of HPLC. The results are summarized in Table 5 below. The OFC was also subjected to a bacteria count; no Enterobacteriaceae were found. The colour of the OFC was determined to be 40 Icumsa units. The conductivity of the OFC, measured after diluting the OFC to a solids content of 28°Bx, was 80 μS/cm.

*Table 5*

| Example number | 7 |
|---|---|
| Non-Fructooligosaccharides | |
| - Fructose | 2.5 |
| - Glucose | 29.5 |
| - Sucrose | 8.7 |
| Fructooligosaccharides | |
| - DP2 (other than Sucrose) | 0.9 |
| - DP3 | 22.6 |
| - DP4 | 28.2 |
| - DP5 and higher | 7.6 |
| $\rightarrow$ Total FOS | 59.2 |

This Example shows that an OFC according to the invention can also be prepared by using Organic sucrose from sugar beet origin. It is noted that in this example 7, the amount of enzyme used, at 0.64 U per gram dry matter of raw material, was rather high when taking into account this specific enzyme and these specific reaction conditions. It is theorised that this high dosage lies at the cause of the circumstance that the weight percentage of FOS having DP3 in the OFC is somewhat lower than in other Examples.

Comparative Experiment B

A sample of a commercially available FC was obtained. The FC was identified as Meioligo W, the supplier of the FC was Meiji Food Materia Co, a Meiji Seika group company. The sample was analysed by means of HPLC; the results are provided in Table 6.

*Table 6*

| Comparative Experiment | B |
|---|---|
| Non-Fructooligosaccharides | |
| - Fructose | 3.8 |
| - Glucose | 28.7 |
| - Sucrose | 11.3 |
| Fructooligosaccharides | |
| - DP2 (other than Sucrose) | 0.8 |
| - DP3 | 27.8 |
| - DP4 | 24.0 |
| - DP5 and higher | 3.5 |
| $\rightarrow$ Total FOS | 56.2 |
| $\rightarrow$ % of FOS having DP3 | 49 |

Comparative Experiment B demonstrates that the amount of Fructooligosaccharides having a DP3 in the commercially available FC is significantly lower than in the FC produced according to the invention.

**Claims**

1.  Process for the production of a fructooligosaccharide (FOS) composition on industrial scale, comprising the steps of:

    a) providing a raw material containing sucrose;
    b) bringing the raw material together with an enzyme to form a mixture, whereby the enzyme has an endo-inulinase activity and is in free, non-immobilized form;
    c) exposing the mixture to conditions whereby FOS-forming can take place for an amount of time such that the FOS in the mixture constitutes at least 45 wt.% of the total amount of carbohydrates in the mixture, thereby forming the FOS composition; wherein the solids content of the mixture lies between 55°Bx and 80°Bx and the temperature lies between 55°C and 76°C;
    d) optionally deactivating the enzyme;
    e) recovering the FOS composition,

    wherein in step a) the amount of processed sucrose lies between 500 and 500,000 kg per 24 hours.

2.  Process according to claim 1, wherein in none of the steps use is made of one or more of the following substances: anti-scaling agent, anti-foaming agent, bactericide, biocide, and flocculant.

3.  Process according to claim 1 or 2, wherein in none of the steps use is made of an ion exchange resin.

4.  Process according to any one of claims 1 - 3, wherein the enzyme is not made by a genetically modified organism.

5.  Process according to any one of claims 1 - 4, wherein in step c) the conditions whereby FOS-forming can take place include a temperature lying within the range of 55°C - 75°C and a solids content lying between 55°Bx and 80°Bx, and wherein the amount of time the mixture is exposed to these conditions is such that at least 50 wt.% of the carbohydrates in the mixture are FOS.

6.  Process according to claim 5, wherein the temperature in step c) lies between 60°C and 72°C and the solids content lies between 61 and 75°Bx.


**Patentansprüche**

1.  Verfahren zum Herstellen einer Fructooligosaccharid(FOS)-Zusammensetzung auf industriellem Maßstab, umfassend die Schritte:

    a) Bereitstellen eines Ausgangsmaterials, das Saccharose enthält;
    b) Zusammenbringen des Ausgangsmaterials mit einem Enzym, um ein Gemisch zu bilden, wobei das Enzym eine Endoinulinase-Aktivität aufweist und in freier, nicht-immobilisierter Form vorliegt;
    c) Exponieren des Gemischs gegenüber Bedingungen, bei denen FOS-Entstehung erfolgen kann, über einen solchen Zeitraum, dass das FOS in dem Gemisch wenigstens 45 Gew.-% der Gesamtmenge an Kohlenhydraten in dem Gemisch bildet, um so die FOS-Zusammensetzung zu bilden; wobei der Feststoffgehalt des Gemischs zwischen 55 °Bx und 80 °Bx liegt und die Temperatur zwischen 55 °C und 76 °C liegt;
    d) gegebenenfalls Deaktivieren des Enzyms;
    e) Gewinnen der FOS-Zusammensetzung,

    wobei bei Schritt a) die Menge an verarbeiteter Saccharose zwischen 500 und 500.000 kg pro 24 Stunden liegt.

2.  Verfahren gemäß Anspruch 1, wobei bei keinem der Schritte einer oder mehrere der folgenden Stoffe verwendet wird: Antiablagerungsmittel, Antischaummittel, Bakterizid, Biozid und Flockungsmittel.

3.  Verfahren gemäß Anspruch 1 oder 2, wobei bei keinem der Schritte ein Ionenaustauschharz verwendet wird.

4.  Verfahren gemäß einem der Ansprüche 1-3, wobei das Enzym nicht durch einen genetisch modifizierten Organismus hergestellt wird.

**5.** Verfahren gemäß einem der Ansprüche 1-4, wobei bei Schritt c) die Bedingungen, bei denen FOS-Entstehung erfolgen kann, eine Temperatur, die in dem Bereich von 55 °C bis 75 °C liegt, und einen Feststoffgehalt, der zwischen 55 °Bx und 80 °Bx liegt, umfassen, und wobei der Zeitraum, über den das Gemisch gegenüber diesen Bedingungen exponiert wird, so ist, dass wenigstens 50 Gew.-% der Kohlenhydrate in dem Gemisch FOS sind.

**6.** Verfahren gemäß Anspruch 5, wobei die Temperatur bei Schritt c) zwischen 60 °C und 72 °C liegt und der Feststoffgehalt zwischen 61 und 75 °Bx liegt.

**Revendications**

**1.** Procédé de production d'une composition de fructooligosaccharide (FOS) à l'échelle industrielle, comprenant les étapes consistant à :

a) fournir une matière première contenant du saccharose ;
b) mettre en contact la matière première avec une enzyme afin de former un mélange, où l'enzyme possède une activité d'endo-inulinase et se trouve sous une forme libre non immobilisée ;
c) exposer le mélange à des conditions où la formation de FOS peut avoir lieu pendant une quantité de temps telle que le FOS dans le mélange constitue au moins 45% en poids de la quantité totale de glucides dans le mélange, formant ainsi la composition de FOS ; dans lequel la teneur en matières solides du mélange se trouve entre 55°Bx et 80°Bx et la température se trouve entre 55°C et 76°C ;
d) éventuellement désactiver l'enzyme ;
e) récupérer la composition de FOS,

où, dans l'étape a), la quantité de saccharose traité se trouve entre 500 et 500 000 kg par 24 heures.

**2.** Procédé selon la revendication 1, dans lequel on ne fait pas usage, dans aucune des étapes, d'une ou plusieurs parmi les substances suivantes : un agent antitartre, un agent antimousse, un bactéricide, un biocide et un floculant.

**3.** Procédé selon la revendication 1 ou 2, dans lequel on ne fait pas usage, dans aucune des étapes, d'une résine d'échange d'ions.

**4.** Procédé selon l'une quelconque des revendications 1-3, dans lequel l'enzyme n'est pas fabriquée par un organisme génétiquement modifié.

**5.** Procédé selon l'une quelconque des revendications 1-4, dans lequel, dans l'étape c), les conditions où la formation de FOS peut avoir lieu comportent une température se trouvant dans la plage de 55°C-75°C et une teneur en matières solides se trouvant entre 55°Bx et 80°Bx, et où la quantité de temps pendant laquelle le mélange est exposé à ces conditions est telle qu'au moins 50% en poids des glucides dans le mélange sont constitués de FOS.

**6.** Procédé selon la revendication 5, dans lequel la température dans l'étape c) se trouve entre 60°C et 72°C et la teneur en matières solides se trouve entre 61 et 75°Bx.

**EP 2 294 093 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62295299 A
- JP 1137974 A
- EP 0889134 A

**Non-patent literature cited in the description**

- **BARRIE E. NORMAN ; BIRGITTE HØJER-PEDER-SEN.** The Production of Fructooligosaccharides from Inulin or Sucrose Using Inulinase or Fructosyltransferase from Aspergillus ficuum. *Denpun Kagaku,* 1989, vol. 36 (2), 103-111
- **ANDRELINA M.P. SANTOS ; FRANCISCO MAUGERI.** Synthesis of Fructooligosaccharides from Sucrose Using Inulinase from Kluyveromyces marxianus. *Food Technol. Biotechnol.,* 2007, vol. 45 (2), 181-186
- **CSANADI, ZS. ; SISAK, CS.** Immobilization of pectinex ultra SP-L pectinase and its application to production of fructooligosaccharides. *Acta Alimentaria,* 2006, vol. 35 (2), 205-212